# EUROPEAN PATENT SPECIFICATION

(11) **EP 1 623 662 B1**
(45) Date of publication and mention of the grant of the patent: **17.06.2009**
(21) Application number: 04730704.6
(22) Date of filing: 30.04.2004
(51) Int. Cl.: A61B 1/015

(54) **ENDOSCOPE FLUID CONTROL INPUT DEVICE**
ENDOSKOP-FLUID-KONTROLL-EINGABEVORRICHTUNG
DISPOSITIF D'ENTREE DE REGULATION D'UN FLUIDE ENDOSCOPIQUE

(30) Priority: 12.05.2003 JP 2003133705
(43) Date of publication of application: 08.02.2006
(60) Divisional application: 08009381.8
(73) Proprietor: Olympus Corporation, Tokyo 151-0072 (JP)
(72) Inventor: ARAI, Keiichi, c/o Olympus Corporation, Shibuya-ku, Tokyo 1510072 (JP); MAEDA, Toshinari c/o Olympus Corporation, Shibuya-ku, Tokyo 151-0072 (JP); IKEDA, Yuichi c/o Olympus Corporation, Shibuya-ku, Tokyo 151-0072 (JP); MIYAGI, Takayasu c/o Olympus Corporation, Shibuya-ku, Tokyo 151-0072 (JP)
(74) Representative: von Hellfeld, Axel
(86) International application number: PCT/JP2004/006312
(87) International publication number: WO 2004/098394

(56) References cited:
- WO-A-98/02894
- GB-A- 2 335 080
- JP-A- 8 299 263
- JP-A- 11 032 979
- JP-A- 2000 332 427
- JP-U- 2 135 002
- US-A- 5 872 527
- US-A- 5 938 589
- US-B1- 6 303 887

## Description

### Technical Field

The present invention relates to a fluid control input device for endoscope, and more particularly, to a fluid control input device for endoscope applied to a fluid control apparatus for endoscope, which supplies the fluid by using a channel arranged in an endoscope and absorbs the intracavital tissue, serving as an operating member upon controlling the supply and absorption of the fluid.

A fluid control input device in accordance with the preamble of the claim 1 is known from US-A-5,938,589. Also US-B1-6,303,887 discloses a corresponding device.

### Background Art

Recently, an endoscope has been widely used in the medical field. In the endoscope used for the medical field, when an endoscope inserting unit is inserted in the body cavity, the intracavital body fluid, dirt, and mucus are necessarily adhered to an observing window arranged to the distal end of the inserting unit, and the observation via the observing window is thus not sufficiently performed.

Therefore, usually, a conventional general endoscope has an air-supply and water-supply function, by which an operating button arranged to an operating unit on the hand side is operated to perform the solution supply operation and the observing window is thus cleaned or the air supply operation is performed to blow off the solution remaining on the surface of the observing window after the cleaning operation. The air supply function is also used for the case of intracavitally supplying the air so as to easily observe a desired observing portion.

Further, the conventional endoscope has an absorbing function, by which the tissue, such as the intracavital target part or diseased part, is partly absorbed and is obtained as specimen. The operation of air supply and water supply and the absorbing operation are performed via an air supplying channel, a water supplying channel, or an absorbing channel arranged in the endoscope.

Japanese Unexamined Patent Application Publication No. 2000-189380 and the like conventionally discloses and proposes various fluid control apparatuses for performing the water supply operation, the air supply operation, and the absorbing operation and operating members (fluid control input devices for endoscope (hereinafter, properly abbreviated to fluid control input devices)) arranged to the operating unit, for operating the fluid control apparatuses in the above-mentioned endoscopes.

In the operating member of the fluid control apparatus in the endoscope apparatus disclosed in Japanese Unexamined Patent Application Publication No. 2000-189380, a user presses-in an operating button, a cylinder which descends in accordance with the pressing operation is arranged to a switch main body, a shading plate is integrally arranged to the bottom of the cylinder, a photo-interrupter is arranged at the shading position in accordance with the descending operation of the shading plate, a reflecting plate is arranged to the bottom surface of the cylinder, predetermined beams are irradiated to the reflecting plate, and a reflecting-type photo-interrupter is arranged at the facing position of the reflecting plate so as to receive reflecting light of the reflecting plate. The output of the reflecting-type photo-interrupter is varied depending on the amount of pressing operation of the operating button.

In the operating member with the above-mentioned structure, the cylinder and the shading plate descend in accordance with the pressing operation of the operating button. Thus, the shading plate shades the reflecting-type photo-interrupter. Then, at the timing for shading the photo-interrupter by the shading plate, the absorbing operation (or air supply or water supply) operation starts. Corresponding to the output of the reflecting-type photo-interrupter in accordance with the amount of pressing operation of the operating button, the amount of absorption (or the amount of air supplied or the amount of water supplied) is controlled. Therefore, only the simple operation for pressing the operating button enables the fine adjustment of the amount of absorption (the amount of air supplied or the amount of water supplied).

In the operating member in the above form, a first-step operation of one operating member controls the on/off operation, and a second-step operation performs the fine adjustment of the amount of operation in the on-state as one example (referred to as a first example).

In another example of the operating member disclosed in Japanese Unexamined Patent Application Publication No. 2000-189380, a first-step operation with a predetermined amount of operation performs the air supply operation, the operation is switched to the water supply operation at a predetermined position, and a second-step and subsequent-step operation performs the water supply operation, as a two-step switch (referred to as a second example).

The operating member in the second example has a switch main body integrally having a cylinder, and the cylinder has, at the top end thereof, an air supply button which is repulsed in the pressing-up direction of a spring member. In the center of the switch main body, a water supply button is arranged, and the water supply button has a hole portion into which the air supply button is inputted upon pressing the air supply button. The water supply button is also repulsed by a spring member in the pressing-up direction to the switch main body.

On the bottom of the cylinder, a shading plate is integrally arranged. At the shading position in accordance with the descending operation of the shading plate, two photo-interrupters for air supply and water supply are laminated. On the bottom surface of the cylinder, a reflecting plate is arranged, predetermined beams are irradiated to the reflecting plate, and a reflecting-type photo-interrupter is arranged at the facing position of the reflecting plate so as to receive the reflecting light of the reflecting plate. The reflecting-type photo-interrupter detects the amount of light received, which changes depending on the amount of pressing operation of the air supply button, thereby controlling the water supply operation.

An example of an operating member of another two-step switch is disclosed as follows (referred to as a third example).

The operating member in the third example has the same structure as that of the operating member in the second example. However, the reflecting plate and the reflecting-type photo-interrupter are not used, the two photo-interrupters laminated in the second example are arranged in parallel, and, corresponding to this, a shading cylinder for shading a photo-interrupter for water supply and a photo-interrupter for air supply is integrally arranged to the cylinder. By setting the length of the shading plate to be varied, the corresponding photo-interrupter is shaded at the varying timing in accordance with the pressing operation of the operating button.

In the operating members in the second and third examples, the start operation and the fine adjustment of the air supply operation are performed in the first operation, and the air supply button comes into contact with the top surface of the water supply button. At the timing for starting to press the water supply button (second operation), the operation is switched from the air supply operation to the water supply operation. And, the water supply button is further pressed-in, thereby controlling the water supply operation. In this case, just before the first operation is switched to the second operation, the air supply operation is performed. At the position just before the switching operation, the amount of air supplied is set to be maximum in the air supply operation. In the second operation, that is, at the maximum pressing position of the water supply button, the amount of water supplied is set to be maximum in the water supply operation.

However, means disclosed in Japanese Unexamined Patent Application Publication No. 2000-189380 does not control a plurality of operations since the one operating member in the first example performs the start of one operation and the fine adjustment thereof.

In the operating members in the second and third examples, the first operation performs the start of the air supply operation and fine adjustment thereof, the air supply button comes into contact with the top surface of the water supply button and the water supply button is pressed. Then, the operation is switched from the air supply operation to the water supply operation, and the one operating member controls and switches the two operations.

However, the timing for switching the air supply operation corresponding to the first operation and the water supply operation corresponding to the second operation is set to a predetermined timing after the air supply button at the first step comes into contact with the water supply button at the second step. The switching timing is not precisely determined. Thus, an unnecessary amount of force is applied to the operating finger in the operation at the maximum position of the air supply operation at the first step and, then, the operation can be undesirably switched to the water supply operation. Therefore, in the case of the air supply operation to expand the stomach, an operator has to pay utmost attention to prevent the excessive pressing operation of the air supply button and simultaneously needs the operation for continuing the half-pressing operation of the operating member (operation for keeping a predetermined position within the range of the first operation). Further, upon erroneously switching to the water supply operation, the desired result in the observation using the endoscope is not obtained.

After the air supply button at the first step comes into contact with the water supply button at the second step, until actually switching operation, for some time, a so-called dead zone without the change in signal is set. Therefore, in accordance with the contact sense of the air supply button and the water supply button, the switching timing of the two operations is notified to the operator. However, the notifying means is not active and the switching timing is not easily recognized.

Further, in the operation for pressing the air supply button at the first step, a zone without generating the signal at the initial operation thereof, that is, the zone until the shading plate first shades the photo-interrupter becomes the so-called dead zone. With the above-mentioned setting, as the amount of pressing operation (operating stroke) of the entire operating member is increased, the dimension of the operating member in the height direction is increased. Thus, the size of the operating member is liable to be increased. When the size of the operating member is increased, an operator having relatively small hands (or relatively short fingers) cannot easily operate the operating member.

Meanwhile, the operating member disclosed in Japanese Unexamined Patent Application Publication No. 2000-189380 has a large number of components, such as springs. The internal mechanism of the operating member is complicated. Thus, when the dirt or stain enters the operating member, the operating member is not cleaned easily.

Then, in order to prevent the intrusion of the dirt or stain, a movable portion of the air supply button and the water supply button has a waterproof seal and the like. However, at the portion having the waterproof seal, the component is always slid in the operation. Therefore, the portion is used for a long time and then the portion is worn and the waterproof ability gradually deteriorates.

The present invention is devised in consideration of the circumstances. It is an object of the present invention to provide a fluid control input device for endoscope, in which the operator easily and certainly recognizes the switching point of two operations or the timing after/before the switching operation with the dust-proof and waterproof structure, even upon using the operating member having the two-step switch.

### Disclosure of Invention

The object of the present invention is to provide a fluid control input device that is reliable in function and that can be handled conveniently with a good operation feeling for the operator.

The fluid control input device in accordance with the present invention is described in claim 1.

Preferred embodiments of the device are described in the dependent claims.

### Brief Description of the Drawings

Fig. 1 is a schematic diagram showing the schematic structure of fluid channels and pipes in a fluid control apparatus connected to an endoscope used for a fluid control input device according to a first embodiment of the present invention;
Fig. 2 is a sectional view showing the detailed structure of the fluid control input device shown in Fig. 1;
Fig. 3 is a diagram showing a change relationship between the amount of pressing force and the amount of air supplied upon pressing the fluid control input device shown in Fig. 1 and the change in amount of pressing force upon switching from the air supply operation to the water supply operation;
Fig. 4 is a sectional view showing a main portion of the structure of a cutout of the components in a fluid control input device according to a modification of the first embodiment of the present invention;
Fig. 5 is an enlarged sectional view showing a main portion of an enlarged periphery of a click mechanism in the fluid control input device shown in Fig. 4 according to the modification;
Fig. 6 is a sectional view showing the detailed structure of a fluid control input device according to a second embodiment of the present invention;
Fig. 7 is a sectional view showing the structure of a fluid control input device not falling under the scope of the invention ;
Fig. 8 is a schematic diagram showing the structure of a fluid control input device according to one modification of the device of fig. 7.
Fig. 9 is a sectional view showing the structure of a fluid control input device according to a third embodiment of the present invention; and

### Best Mode for Carrying Out the Invention

Hereinbelow, embodiments of the present invention will be described with reference to the drawings.

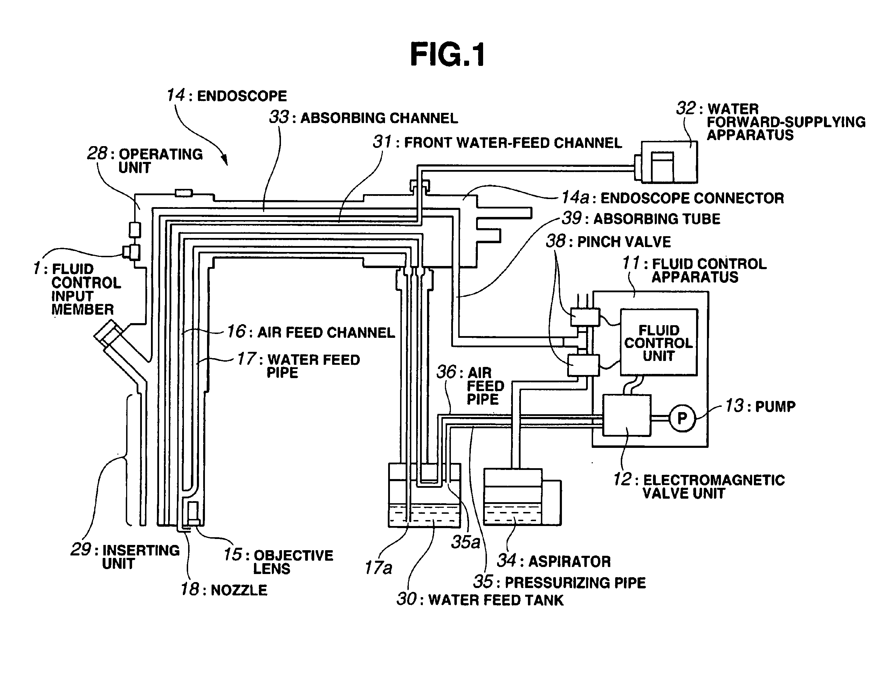

Fig. 1 is a schematic diagram showing the schematic structure of fluid channels and pipes in a fluid control apparatus connected to an endoscope used for a fluid control input device according to the first embodiment of the present invention.

Referring to Fig. 1, an endoscope 14 comprises: an operating unit 28; and an inserting unit 29. In the endoscope 14, an air supplying channel 16, a water supplying channel 17, a water forward-supplying channel 31, and an absorbing channel 33 are formed. The operating unit 28 comprises various operating members, e.g., a fluid control input member 1, serving as a fluid control input device. The inserting unit 29 has, at the distal end thereof, image pickup means (not shown), and has, on the front surface of the distal end thereof, an objective lens 15 and openings of the channels.

The air supplying channel 16 and the water supplying channel 17 are formed to merge to one channel near the distal end of the inserting unit 29. Near the opening of the front surface of the one channel, a nozzle 18 is arranged. The nozzle 18 is shaped to spray a predetermined fluid toward the surface of the objective lens 15, and is arranged at a predetermined position near the objective lens 15.

The water forward-supplying channel 31 is inserted in the inserting unit 29 and the operating unit 28 of the endoscope 14. Further, the water forward-supplying channel 31 is extended from an endoscope connector 14a and thereafter is connected to a water forward-supplying device 32.

Similarly, the water supplying channel 17 is inserted in the inserting unit 29 and the operating unit 28 of the endoscope 14. The water supplying channel 17 is extended from the endoscope connector 14a, and thereafter is connected to a water supply tank 30 for supplying the water to the water supplying channel 17. In this case, a distal opening 17a of the water supplying channel 17 is arranged at a predetermined position to be always sunk in the water pooled in the water supply tank 30.

Similarly, the air supplying channel 16 is inserted in the inserting unit 29 and the operating unit 28 of the endoscope 14. The air supplying channel 16 is extended from the endoscope connector 14a and thereafter is guided to the water supply tank 30. Then, the air supplying channel 16 is connected to an air supplying pipe 36 via the water supply tank 30.

The air supplying pipe 36 is connected to an electromagnetic valve unit 12 arranged in a fluid control apparatus 11. From the electromagnetic valve unit 12, a pressurizing pipe 35 for pressurizing the inside of the water supply tank 30 is connected to the water supply tank 30. In this case, a distal opening 35a of the pressurizing pipe 35 is arranged at a predetermined position where it is opened in the water supply tank 30 and it is not sunk in the water pooled in the water supply tank 30. Incidentally, a pump 13 drives the electromagnetic valve unit 12 of the fluid control apparatus 11.

Incidentally, the electromagnetic valve unit 12 comprises a plurality of electromagnetic valves (individual electromagnetic valve is not shown), e.g., electromagnetic valves corresponding to the use for water supply operation and air supply operation. In accordance therewith, the pump 13 comprises a plurality of pumps (individual pump is not shown), e.g., pumps corresponding to the use for water supply operation and air supply operation. The individual pump drives the corresponding electromagnetic valve.

Further, the absorbing channel 33 is similarly inserted in the inserting unit 29 and the operating unit 28 of the endoscope 14. The absorbing channel 33 is extended from the endoscope connector 14a and thereafter is guided to an aspirator 34 via an absorbing valve 39 and a leak tube inserted to two pinch valves 38 controlled by the fluid control apparatus 11.

Meanwhile, as mentioned above, the operating members are arranged to the operating unit 28 of the endoscope 14. Among the operating members, a predetermined signal line (not shown) for transmitting a signal generated by the fluid control input member 1 (e.g., an output signal of an optical sensor 2, which will be described later) is connected to the fluid control apparatus 11 via the inside of the endoscope 14 and the endoscope connector 14a.

Therefore, the signal outputted from the fluid control input member 1 is transmitted to the electromagnetic valve unit 12 of the fluid control apparatus 11. Thus, among the plurality of electromagnetic valves (not shown) of the electromagnetic valve unit 12, the corresponding electromagnetic value is opened/closed. In accordance therewith, various operations are performed, e.g., the air supply operation starts or ends, the amount of air supplied increases or decreases, the water supply operation starts or ends, and the spray operation starts or ends.

A signal from another predetermined operating member (absorbing operating member) different from the fluid control input member 1 is transmitted from the fluid control apparatus 11 to the two pinch valves 38. Then, in response thereto, the pinch valves 38 opens/closes the absorbing valve 39 and the leak tube and the absorbing operation starts or ends, and the amount of absorption increases/decreases.

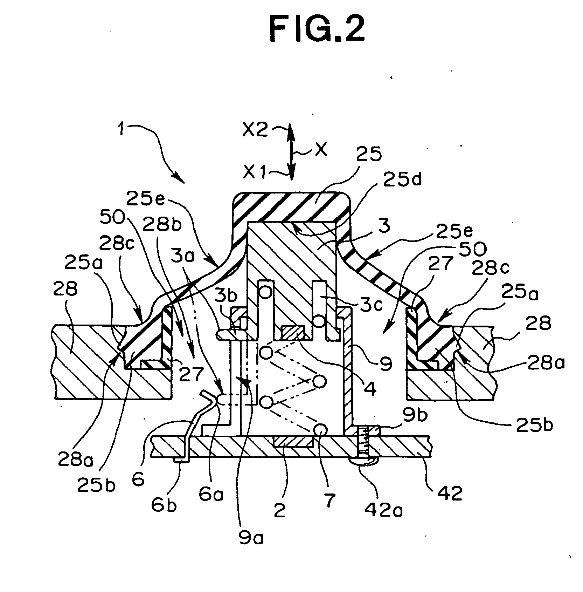

Fig. 2 is a sectional view showing the detailed structure of the fluid control input device (fluid control input member 1) according to the first embodiment.

Referring to Fig. 2, the fluid control input member 1 according to the first embodiment comprises: a rubber cover 25; an operating button 3; a button receiving member 9; a fixing substrate 42; a repulsive spring 7; a click spring 6; a light-emitting device 4; and the optical sensor 2.

The rubber cover 25 is watertight covering means for waterproof operation or a covering member. The rubber cover 25 watertightly covers the entire components, such as the operating button 3, serving as moving means or a moving member, partly forming the fluid control input member 1 and is bent in a predetermined form in accordance with the pressing operation of the operating button 3.

The operating button 3 is moving means that is movably arranged in the direction shown by an arrow X1 in Fig. 2.

The button receiving member 9 supports the operating button 3, and guides the moving direction of the operating button 3.

The button receiving member 9 is fixed to the fixing substrate 42.

The repulsive spring 7 is arranged in the inner space of the button receiving member 9 to be sandwiched between the operating button 3 and the fixing substrate 42, and is position restoring means or a restoring member, containing a coil spring or the like, which repulses the operating button 3 in the direction shown by an arrow X2 shown in Fig. 2.

The click spring 6 partly forms a click mechanism, and is arranged, indicating the switching point of a first-step switch and a second-step switch so as to form the fluid control input member 1 as a two-step switch. As will be described later, the click spring 6 is amount-of-force changing means or an amount-of-force changing mechanism, which generates temporary and sharp change in amount of force.

The light-emitting device 4 is arranged to the bottom surface of the operating button 3, and irradiates predetermined luminous fluxes.

The optical sensor 2 is arranged at a predetermined position facing the light-emitting device 4 on the surface of the fixing substrate 42, and is signal generating means or a detecting member of the amount of operation which receives luminous fluxes from the light-emitting device 4, converts the luminous fluxes into a predetermined electrical signal, and outputs the converted signal.

Preferably, the rubber cover 25 contains a silicon rubber or fluorocarbon rubber with the higher chemical-resistance and repeating bending resistance.

The fixing substrate 42 is a fixing member, such as an electric substrate, and is arranged at a predetermined position in the operating unit 28 (refer to Fig. 1) of the endoscope 14. At a predetermined position of the fixing substrate 42, the button receiving member 9 is fixed by using a fixing member 42a, such as a screw.

As mentioned above, on the surface of the fixing substrate 42, the optical sensor 2 is arranged with the light receiving surface thereof directed to the top. The positioning of the operating button 3 and the button receiving member 9 is set to arrange the irradiating surface of the light-emitting device 4 at the position facing the light receiving surface of the optical sensor 2. That is, the optical sensor 2 is arranged at a predetermined position within the irradiating range of the luminous fluxes from the light-emitting device 4. The positions of the optical sensor 2 and the light-emitting device 4 are set for the optical sensor 2 to receive the luminous fluxes outputted from the light-emitting device 4.

As mentioned above, the repulsive spring 7 constantly repulses the operating button 3 in the direction shown by the arrow X2 shown in Fig. 2. Thus, the resistance of the pressing operation with a predetermined amount of force acts to the operating button 3. An operator presses the operating button 3 in the direction shown by an arrow X1 shown in Fig. 2. Thereafter, the amount of pressing force is reset and then the operating button 3 is repulsed and moved in the direction shown by the arrow X2 shown in Fig. 2. The operating button 3 is finally restored to a predetermined position.

The operating button 3 is a substantially cylindrical member having a flange unit 3b at the bottom end thereof, and has projected portions 3a, serving as engaging portions, which are projected to the outer circumference from the flange unit 3b at three portions with the same interval.

The button receiving member 9 is a substantially cylindrical member having a flange unit 9b at the bottom end, with openings at both ends thereof. A screw hole in which the fixing member 42a is screwed is pierced through the flange unit 9b. Thus, the button receiving member 9 is fixed with a screw to the fixing substrate 42. Three groove notches 9a are formed onto the outer circumference of the button receiving member 9 with the same interval. The groove notches 9a are formed onto the outer circumference of the button receiving member 9, at the periphery ranging from the bottom end to the top end, and are opened to the bottom end and are closed to the top end.

Therefore, the operating button 3 is inserted in the button receiving member 9. Then, the projected portions 3a of the operating button 3 are slidably engaged with the groove notches 9a. Thus, the operating button 3 is moved in the direction shown by an arrow X without rotation. The operating button 3 fit from the bottom side of the button receiving member 9 is not pulled out to the side of the top end. That is, the groove notches 9a have a function of guiding means of the operating button 3, and further have a positioning function at the top end of the operating button 3.

On the other hand, when the operating button 3 is fit into the button receiving member 9, the repulsive spring 7 is arranged in the button receiving member 9. In this case, on the side of the bottom end of the operating button 3, a circular groove portion 3c for fitting the top end of the repulsive spring 7 like a coil is pierced. Therefore, the repulsive spring 7 is positioned in the button receiving member 9. In this state, the button receiving member 9 is fixed to the fixing substrate 42, thereby operating the repulsive force of the repulsive spring 7 in the extending direction from the bottom end of the operating button 3. The operating button 3 is always repulsed in the direction shown by the arrow X2 shown in Fig. 2.

When the projected portions 3a of the operating button 3 are fit into the groove notches 9a of the button receiving member 9, the projected portions 3a are set to be slightly projected to the outside from the outer circumference of the button receiving member 9.

At positions corresponding to the three projected portions 3a, the click springs 6 which are formed by bending a plate spring or the like are arranged. The click springs 6 have fixing ends 6b at one end thereof which are fixed at predetermined positions of the fixing substrate 42. Further, the click springs 6 have free ends 6a at the other end thereof which are slightly projected on the locus for moving the projected portions 3a.

Therefore, the operating button 3 is moved in the X direction and then the projected portions 3a come into contact with the free ends 6a of the click springs 6 at predetermined positions (refer to reference numeral 3a at the position shown by a dotted line in Fig. 2). In this case, the projected portions 3a of the operating button 3 press the click springs 6 against the repulsive force of the click springs 6 and are moved in the direction shown by the arrow X. When the projected portions 3a go over the click spring 6, the predetermined sense of click operation is generated. The sense of click operation is generated just before switching the first-step operation and the second-step operation of the fluid control input member 1. As mentioned above, the click spring 6 and the projected portions 3a form the click mechanism, serving as amount-of-force changing means or an amount-of-force changing mechanism, which generates the temporary and sharp change in amount of force.

The optical sensor 2 receives the luminous fluxes from the light-emitting device 4, converts the received luminous fluxes into a predetermined electrical signal, and thereafter outputs the electrical signal. In this case, the output signal of the optical sensor 2 changes, depending on the strength of luminous fluxes received. In accordance with the movement of the operating button 3 in the direction shown by the arrow X, the light-emitting device 4 is moved in the same moving direction. Therefore, the strength of luminous fluxes received by the optical sensor 2 changes, depending on a separate distance between the light-emitting device 4 and the optical sensor 2.

Therefore, the output signal of the optical sensor 2 changes, depending on the change in the position corresponding to the amount of pressing force of the operating button 3 having the light-emitting device 4. Thus, the light-emitting device 4 and the optical sensor 2 have, as one set, a function of position detecting means which detects the position or movement of the operating button 3.

As mentioned above, the output signal of the optical sensor 2 is transmitted to the fluid control apparatus 11 via a predetermined signal line. Therefore, the fluid control apparatus 11 receives the signal outputted from the optical sensor 2, and detects the position or the amount of movement of the operating button 3 based on the strength in received signal. The fluid control apparatus 11 controls the pump 13 and the electromagnetic valve unit 12 based on the detecting result to control the operation of a plurality of electromagnetic valves, thereby controlling the switching of a fluid (air and water) supplied to the endoscope 14 or the amount of fluid flow.

The operating button 3 is arranged to be externally projected from a hole portion 28b pierced at a predetermined position of the operating unit 28 in the endoscope 14. The rubber cover 25 containing an elastic modifying member is arranged so as to cover the hole portion 28b.

In this case, a ring rubber insert 27 having the L-shaped cross-section is adhered and fixed to the inner circumference of the hole portion 28b of the operating unit 28. A groove portion 28c is formed to the inner circumference of the hole portion 28b of the operating unit 28. An end 25b of the rubber cover 25 is fit and adhered to the groove portion 28c.

A circumferential groove 28a is formed to the inner wall surface of the hole portion 28b. Corresponding to this, a projected portion 25a circumferentially-formed is formed to the outer circumference of the end 25b of the rubber cover 25. When the rubber cover 25 is fit to the groove portion 28c of the operating unit 28, the circumferential groove 28a of the operating unit 28 is fit into the projected portion 25a with the tightening degree for flatten the projected portion 25a of the rubber cover 25. Thus, a dust-proof and waterproof structure is obtained in the hole portion 28b of the operating unit 28. The entire components of the fluid control input member 1 are watertightly covered with the rubber cover 25.

An inside surface 25d of the top of the rubber cover 25 is adhered and fixed to the top surface of the operating button 3. The rubber cover 25 has a smooth curved portion at the portion facing the portion (shown by reference numeral 25e) ranging from the top surface of the operating button 3 to the side surface. Further, as mentioned above, the end 25b of the rubber cover 25 is fit and adhered to a predetermined position (groove portion 28c) of the operating unit 28.

Thus, in accordance with the pressing operation of the operating button 3, the curved portion 25e is folded into a space 50 between the button receiving member 9 and the hole portion 28b and then the rubber cover 25 is bent in a predetermined form. In the process of the bending, the temporary and sharp change in amount of force is generated. The timing for generating the temporary and sharp change in amount of force of the rubber cover 25 is set at a predetermined timing after/before the generating timing corresponding to the generating timing of a predetermined signal outputted from the optical sensor 2 in accordance with the positional change in the operating button 3. The timing for generating the change in amount of force is set to the same timing for generating the signal, and the timing for generating the change in amount of force can be arbitrarily set.

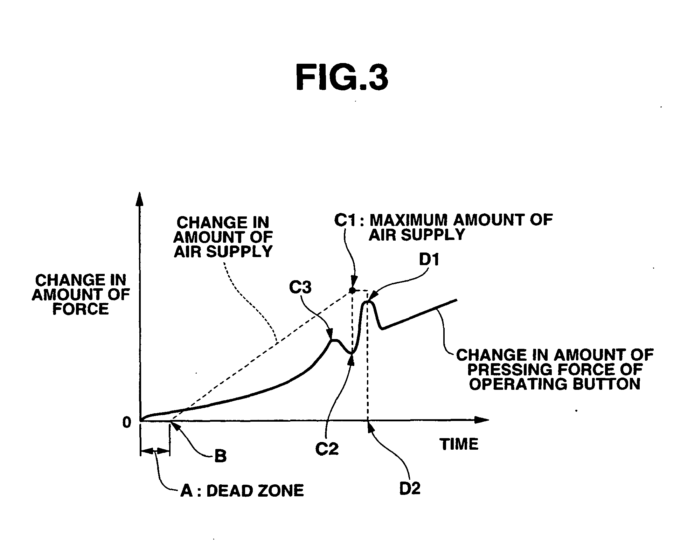

The operation upon operating the fluid control input member 1 with the above-mentioned structure is as follows. Incidentally, Fig. 3 is a diagram showing a change relationship between the amount of pressing force and the amount of air supplied upon pressing the operating button 3 and the change in amount of pressing force upon switching from the air supply operation to the water supply operation.

First, the operator presses the top surface of the operating button 3 from the rubber cover 25 in the direction shown by the arrow X1, thereby moving the operating button 3 in the same direction. In this case, the light emission of the light-emitting device 4 starts. The light emission starts upon starting a main power supply of the endoscope system using the endoscope 14. Alternatively, in response to the start of movement of the operating button 3, the light emission may start. In this case, predetermined means for detecting the movement of the operating button 3 is provided.

In response to the start of light emission of the light-emitting device 4, the optical sensor 2 operates and then the optical sensor 2 receives the luminous fluxes from the light-emitting device 4 and outputs a predetermined signal (information indicating that the operating button 3 operates and a signal indicating positional information on the operating button 3) to the fluid control apparatus 11. In response to this, the fluid control apparatus 11 controls the pump 13 and the electromagnetic valve unit 12, thereby driving a predetermined electromagnetic valve (electromagnetic valve for air supply, not shown). Then, the air supply operation for starting to supply the air in the body cavity having the endoscope 14 via the air supplying pipe 36 and the air supplying channel 16 starts (refer to reference numeral B shown Fig. 3).

Therefore, a predetermined time lag exists during the period from starting the pressing operation of the operating button 3 to actually starting the air supply operation. The time lag is a dead zone shown by reference numeral A in Fig. 3. Incidentally, the time of the dead zone A is arbitrarily set. For example, when the time of the dead zone A is set to zero, even if the fluid control input member 1 is touched at the undesired timing, the air supply operation promptly starts. In order to prevent such a situation, preferably, a predetermined time of the dead zone A is set. When the dead zone A is long, the amount of pressing operation (operating stroke) of the operating button 3 increases. Then, the size of the fluid control input member 1 increases and therefore the dead zone A needs to be properly set in consideration of this.

In accordance with the amount of pressing operation of the operating button 3, the output signal of the optical sensor 2 changes. In accordance therewith, the fluid control apparatus 11 controls the operation for increasing the output of a pump for air supply in the pump 13. Further, in accordance with this, the degree of opening of the corresponding electromagnetic valve for air supply increases, and the amount of air supplied increases as shown by a dotted line in Fig. 3.

Here, upon pressing the operating button 3 in the same direction, the restoring force of the repulsive spring 7 gradually increases in accordance with the amount of pressing operation. The curvature of the smooth curved portion (25e) formed to the rubber cover 25 gradually reduces. In accordance with this, the elastic restoring force of the rubber cover 25 is generated. Therefore, the amount of pressing force of the operating button 3 needs to be increased (refer to Fig. 3).

In accordance with the movement of the operating button 3, the light-emitting device 4 is moved in the same direction. The space between the light-emitting device 4 and the optical sensor 2 gets narrow. Thus, the strength of luminous fluxes received by the optical sensor 2 increases. Therefore, as mentioned above, the output signal of the optical sensor 2 changes depending on the amount of pressing operation of the operating button 3.

Thus, upon pressing the operating button 3, at the timing at which the amount of air supplied is maximum (refer to reference numeral C1 in Fig. 3), the bending of the rubber cover 25 is maximum and thereafter a predetermined portion (curved portion 25e) of the rubber cover 25 is folded in the direction (approximately parallel direction) along the moving direction (direction shown by the arrow X) of the operating button 3 toward the hole portion 28b. That is, in this state, the predetermined portion (curved portion 25e) of the rubber cover 25 is accommodated in a predetermined space of the hole portion 28b (refer to reference numeral C2 in Fig. 3).

In the state just before the above state, that is, just before the state in which the predetermined portion (curved portion 25e) of the rubber cover 25 is folded, the amount of force obtained by adding the amount of elastic restoring force of the rubber cover 25 to the amount of restoring force of the repulsive spring 7 (both the force is repulsive force in the direction shown by the arrow X2) operates, as the resistant sense against the finger of the operator which presses the operating button 3 (refer to reference numeral C3). Thereafter, at the timing at which the predetermined portion (curved portion 25e) of the rubber cover 25 is bent and is folded, the amount of force in the direction shown by the arrow X1 temporarily and sharply operates (refer to reference numeral C2).

Therefore, at the moment, the resistant sense against the finger of the operator temporarily and sharply reduces, thereby generating so-called sense of click operation. Thus, the operator clearly recognizes the fact. That is, the operator recognizes that the rubber cover 25 is bent and is folded, thereby recognizing that the amount of air supplied is set to be maximum.

In this state, the pressing operation of the operating button 3 stops and the state keeps. Then, the air supply operation continues while keeping the maximum amount of air supplied. During this, the operator performs the intracavital observation, examination, and treatment.

In the halfway of the above operation, when the dirt and the like is adhered to the surface of the objective lens 15 arranged to the endoscope 14, the operator performs the water supply operation, thereby cleaning the surface of the objective lens 15.

In order to clean the objective lens 15, the operating button 3 is further pressed from the state. Then, the projected portions 3a of the flange unit 3b of the operating button 3 come into contact with the click springs 6 (refer to the position 3a shown by the dotted line in Fig. 2). Further, the operating button 3 is pressed and then the repulsive force of the click spring 6 operates to the operating button 3. A large amount of pressing force of the operating button 3 is required. When the projected portions 3a go over the click springs 6 (refer to reference numeral D1 in Fig. 3), the amount of pressing force sharply reduces. Thus, the operator obtains the sense of click operation.

In response to the output of the optical sensor 2 just after the timing (reference numeral D), the fluid control apparatus 11 performs the switching control processing from the air supply operation to the water supply operation (refer to reference numeral D2 in Fig. 3). That is, the fluid control apparatus 11 controls the pump 13 and the electromagnetic valve unit 12, thereby driving a predetermined electromagnetic valve (electromagnetic valve for water supply, not shown). Then, the air supply operation to the pressurizing pipe 35 pressurizes the inside of the water supply tank 30, thereby starting the water supply operation for supplying the water (solution) in the water supply tank 30 to the body cavity having the endoscope 14 via the water supplying channel 17.

Therefore, the operator presses the operating button 3 with the amount of force against the repulsive force of the click springs 6, and obtains the sense of click operation when the operating button 3 goes over the click springs 6. After that, the operator recognizes the start of water supply operation.

In other words, the fluid control input device 1 according to the first embodiment is notifying means which sends, to the operator, a notification indicating that the switching operation from the air supply operation to the water supply operation is performed by generating a predetermined signal from the optical sensor 2, by using the change in amount of force (the sense of click operation) generated by the amount-of-force changing means (amount-of-force changing mechanism) (click mechanism) including the click springs 6 and the projected portions 3a.

In the state in which the operation switched to the water supply operation, the surface of the objective lens 15 is cleaned or the like.

After executing the water supply operation and cleaning the surface of the objective lens 15, the amount of pressing force of the operating button 3 is reduced, the restoring force obtained by adding the elastic force of the repulsive spring 7 and the elastic restoring force of the rubber cover 25 operates to the operating button 3, and then the operating button 3 is pressed-up in the direction shown by the arrow X2 shown in Fig. 2. Thus, the amount of water supplied is gradually reduced. In response to the output signal from the optical sensor 2 at a predetermined timing (reference numeral D2) just before the projected portions 3a go over the click springs 6, the fluid control apparatus 11 is switched from the fluid operation to the air supply operation, thereby performing predetermined control processing for spraying the remaining water in the merged channel of the air supplying channel and the water supplying channel on the back side of a nozzle.

Further, the amount of pressing force of the operating button 3 is reduced, thereby gradually reducing the amount of air supplied. The folding state of the rubber cover 25 is restored to the original state shown in Fig. 2. Even in this state, the elastic force of the repulsive spring 7 in the direction shown by the arrow X2 operates to the operating button 3, thereby continuously moving the operating button 3 in the same direction. The operating button 3 is pressed and returned and is positioned at a predetermined point until the flange unit 3b comes into contact with an end of the groove notch 9a of the button receiving member 9 (refer to reference numeral 3b shown by a solid line in Fig. 2). At the timing just before the above state (refer to reference numeral B), the air supply operation ends.

As mentioned above, according to the first embodiment, the sense of click operation generated by the operation when the rubber cover 25 is bent and is folded in accordance with the pressing-down operation of the operating button 3 and the sense of click operation generated when the projected portions 3a go over the click springs 6 are clearly transmitted to the finger of the operator. At a predetermined timing after the sense of click operation is generated, the operation is switched from the air supply operation to the water supply operation.

That is, at a predetermined timing in the processing for pressing-down the operating button 3, the fluid control apparatus 11 switches the operation from the air supply operation to the water supply operation. The operator certainly recognizes the switching timing.

Therefore, although the two-step switch executes the two operations including the air supply operation and the water supply operation by using one operating member and the two-step switch forms the fluid control input member 1, the desired operation is executed without switching the two operations by the erroneous operation and the preferable operability is ensured.

Since the user clearly recognizes the timing at which the amount of air supplied is maximum, upon continuing the air supply operation, the amount of pressing operation of the operating button 3, that is, the target of the amount of air supplied is easily estimated. Therefore, the preferable operability is ensured.

Further, the two-step switch structure with the simple structure is realized and this contributes to the reduction in the number of parts and in manufacturing costs. The switching operation of the first step and the second step of the two-step switch electrically detects the position in the noncontact state by using the light-emitting device 4 and the optical sensor 2. Therefore, the structure is highly durable, as compared with the structure using an electrical switch having a conventional electrical contact or the like.

Since the so-called dead zone does not need to be set before/after the switching timing of the operation at the first step (air supply operation) and the operation at the second step (water supply operation), the entire pressing process (distance stroke) of the operating button 3 is reduced and therefore the dimension of the operating member in the height direction is suppressed. Therefore, this contributes to the reduction in size of the endoscope using the fluid control input member 1.

In the conventional two-step switch, the amount of pressing force in the first step and the amount of folding force in the second step change. Thus, a repulsive spring contributing to the operation in the first step and a repulsive spring contributing to the operation in the second step are individually arranged. Further, means for changing the amounts of repulsive force of both the repulsive springs can be provided by using the repulsive spring in the second step containing a wire with a thick diameter or using two repulsive members with different spring constants. However, in this case, the repulsive member with the large amount of repulsive force results in the deterioration in operability. Then, according to the first embodiment, the repulsive spring 7 may be one member which can be operated with the same amount of force in the first step and the operation in the second step. Hence, the preferable operability is easily ensured, and the simple structure of components contributes to the reduction in manufacturing costs.

In the general case, an elastic member, such as a rubber cover, is arranged to the portion which the operator directly touches, such as the operating member. Then, as a buffer material against the amount of force transmitted to the finger of the operator, the rubber cover operates. Therefore, it is well-known that the operator cannot recognize the slight change in amount of force of the repulsive spring generated at the switching point or contact sense. However, in the fluid control input member 1 according to the first embodiment, in the processing for pressing-down the rubber cover 25 together with the operating button 3, the rubber cover 25 is bent. Therefore, the operator clearly recognizes the switching point of the operation.

Further, in the fluid control input member 1 according to the first embodiment, the rubber cover 25 entirely covers the components in the fluid control input member 1. Thus, the amount of pressing force of the operating button 3 is reduced and the dust-proof and waterproof structure is formed by using the rubber cover 25. An operating portion of the operating button 3 does not need the waterproof seal. This contributes to the reduction in components and further to the reduction in manufacturing costs. Simultaneously, the seal member is not provided and there is no danger of the wear of seal member. The high resistance is ensured and the repeating use is possible.

Incidentally, the fluid control input member 1 according to the first embodiment uses the optical sensor 2 and the light-emitting device 4, as position detecting means that detects the position of the operating button 3. However, the present invention is not limited to this position detecting means. For example, another position detecting means including a magnetic member and a magnetic sensor may be used.

When the rubber cover 25 is bent and is folded, the sense of click operation is generated. Then, just after the sense of click operation is generated when the projected portions 3a of the operating button 3 go over the click springs 6, the operation is switched from the air supply operation to the water supply operation. The switching timing is not limited to this. For example, the operation may be switched simultaneously with the timing at which the projected portions 3a go over the click springs 6.

Incidentally, according to the first embodiment, in accordance with the output signal from the optical sensor 2 at the timing just after the sense of click operation is generated when the projected portions 3a go over the click springs 6, the fluid control apparatus 11 performs predetermined control processing for switching the operation from the fluid operation to the air supply operation. In addition, the click spring 6 may be used as an operation change-over switch. In this case, the click springs 6 are moved by the projected portions 3a, thereby generating a predetermined signal. The signal is transmitted to the fluid control apparatus 11. In response to the signal, the fluid control apparatus 11 performs predetermined operation switching control processing. Thus, the same advantages as those according to the first embodiment are obtained.

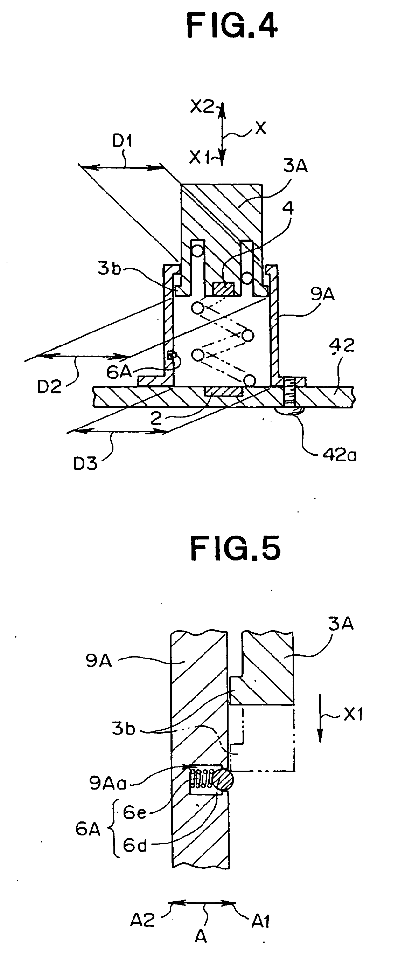

According to the first embodiment, as mentioned above, the switching point of the first step and the second step forming the two-step switch structure using the click spring 6 is shown. However, the present invention is not limited to the above structure. For example, the structure shown in Fig. 4 may be used and the same structure can be obtained.

Fig. 4 is a sectional view showing a main portion of the structure of partly cut-off components in a fluid control apparatus according to one modification of the first embodiment of the present invention.

The structure of a fluid control input member (1), serving as a fluid control input device according to the modification is basically the same as that according to the first embodiment. Only the structure of a click mechanism forming the two-step switch structure is different from that according to the first embodiment. Therefore, referring to Fig. 4, only different portions are described and the remaining portions are not described in detail with reference to the description using Fig. 2.

Referring to Fig. 4, the flange unit 3b, serving as an engaging portion of an operating button 3A of the fluid control input member (1), according to the modification has a smaller amount of projection, as compared with that of the flange unit 3b of the operating button 3 in the fluid control input member 1 according to the first embodiment. In this case, a diameter D2 of the outermost-circumference of the flange unit 3b is pierced through the top surface of the button receiving member 9, and is set so that it is slightly larger than a diameter D1 of a hole portion on the inner circumference, to which the operating button 3A is projected and is substantially equal to or slightly smaller than a diameter D3 (diameter of the hole portion pierced through the bottom surface of the button receiving member 9) of the inner-circumferential wall surface of the button receiving member 9.

The operating button 3 is inserted in the button receiving member 9A and the button receiving member 9A supports the movement of the operating button 3 in the direction shown by an arrow X. The button receiving member 9A is formed by omitting the groove notch 9a according to the first embodiment. That is, the button receiving member 9 has hole portions with diameters D1 and D3 and predetermined shapes on the top surface and the bottom surface thereof. On the side of the lower end (side of the fixing substrate 42) of the button receiving member 9, the button receiving member 9A has the flange unit 9b for fixing itself to the fixing substrate 42. Thus, the button receiving member 9A is fixed and supported to the top surface of the fixing substrate 42 by using the same means (fixing member 42a, such as a screw) as that according to the first embodiment.

In place of the click springs 6 forming the click mechanism according to the first embodiment, at a predetermined position of the inner wall surface of the button receiving member 9A, a click member 6A is arranged, including a ball plunger including a ball 6d and a coil spring 6e.

Fig. 5 is an enlarged sectional view showing a main portion of an enlarged periphery of a click mechanism in the fluid control input device shown in Fig. 4 according to the modification.

Referring to Fig. 5, the click member 6A forming the click mechanism comprises the ball 6d and the coil spring 6e for repulsing the ball 6d. The ball 6d and the coil spring 6e are arranged in hole portions 9Aa arranged at predetermined plural positions of the inner wall surface of the button receiving member 9A. That is, the coil spring 6e is fixed to the bottom surface of the portion 9Aa, and the coil spring 6e repulses the ball 6d in the direction shown by an arrow A1 in Fig. 5. In this case, the diameter of the opening of the portion 9Aa is slightly smaller than the diameter of the ball 6d. Therefore, the fall of the ball 6d from the portion 9Aa to the outside due to the coil spring 6e is prevented. The ball 6d repulsed in the direction shown by the arrow A1 by the coil spring 6e is partly projected to the outside from the opening with a predetermined amount.

In this state, the operator presses the operating button 3A, thereby moving the flange unit 3b of the operating button 3A in the direction shown by the arrow X1 in Fig. 5. Then, the operating button 3A comes into contact with the ball 6d as shown by a dotted line in Fig. 5.

Further, the operating button 3A is pressed and then the ball 6d is moved against the repulsive force of coil spring 6e in the direction of the inside of the hole portion 9Aa, that is, in the direction shown by an arrow A2 in Fig. 5. In this case, the repulsive force of the coil spring 6e operates to the flange unit 3b of the operating button 3A. Thus, the amount of pressing force of the operating button 3A needs to be slightly increased.

The flange unit 3b of the operating button 3A passes through the position facing the hole portion 9Aa (the ball 6d). The repulsive force of the coil spring 6e returns the ball 6d to the original position. Then, the repulsive force of the coil spring 6e to the operating button 3A is sharply released. Therefore, the operator of the operating button 3A obtains predetermined sense of click operation.

When the operator releases the amount of pressing force to the operating button 3A to return the operating button 3A to the original position, similarly the sense of click operation is obtained.

As mentioned above, according to the modification, the same advantages as those according to the first embodiment are obtained.

Next, a description is given of a fluid control input device according to a second embodiment of the present invention.

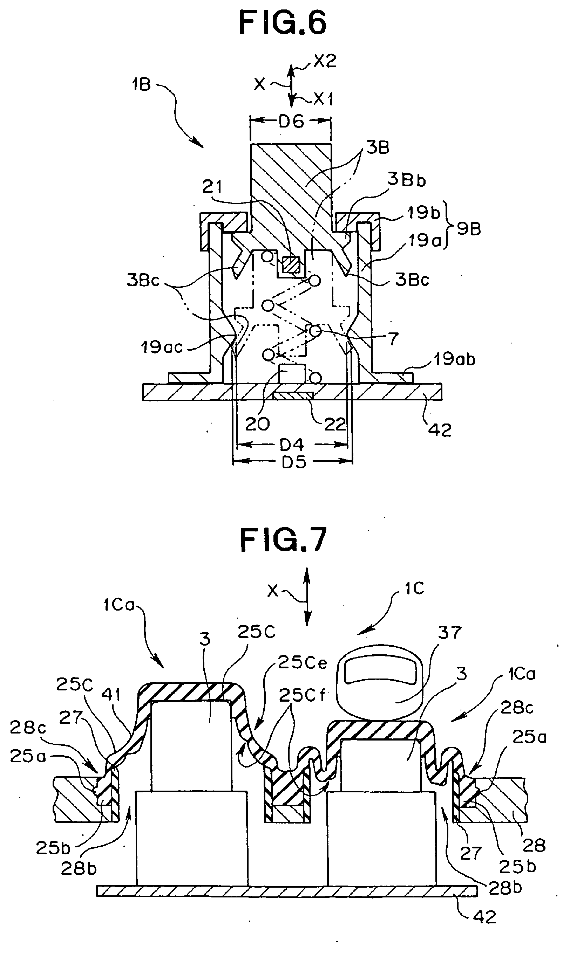

Fig. 6 is a sectional view showing the detailed structure of a fluid control input device (fluid control input member 1B) according to the second embodiment of the present invention.

The structure according to the second embodiment basically uses the structure according to the first embodiment. However, the structures of the operating button, the button receiving member for receiving the operating button, and the click mechanism are different from those according to the first embodiment. Therefore, the same components as those according to the first embodiment are designated by the same reference numerals and a detailed description thereof is omitted. According to the second embodiment, the rubber cover for entirely covering the fluid control input device is arranged. The rubber cover is arranged similarly to that according to the first embodiment, and the drawing is not shown for the purpose of simple drawing. An endoscope using the fluid control input device according to the second embodiment and a fluid control apparatus connected to the endoscope are the same as those according to the first embodiment, therefore, are referred to Fig. 1, and a drawing and a description thereof are not shown.

Referring to Fig. 6, the fluid control input member 1B, serving as a fluid control input device, according to the second embodiment comprises: a waterproof rubber cover (not shown, refer to Fig. 2); the operating button 3B that is arranged movably in the direction shown by an arrow X in Fig. 6; the button receiving member 9B that supports the operating button 3 and guides the moving direction; the fixing substrate 42 to which the button receiving member 9B is fixed; the coil-shaped repulsive spring 7 that is arranged in the inner space of the button receiving member 9B and repulses the operating button 3B in the direction shown by the arrow X2 in Fig. 6; a magnetic member 21, such as a magnet, which is arranged near the bottom end of the operating button 3B; a magnetic sensor 22, serving as signal generating means, which is arranged at a predetermined position facing the operating button 3B on the surface of the fixing substrate 42; and a micro switch 20.

The button receiving member 9B has the structure including two members of a supporting portion 19a and a flange cover 19b. The supporting portion 19a is a substantially cylindrical member having openings at both end surfaces thereof. On one end surface of the supporting portion 19a, the flange unit 19ab is formed. Via the flange unit 19ab, the button receiving member 9B is fixed onto the surface of the fixing substrate 42. As means for fixing the button receiving member 9B to the fixing substrate 42, the means according to the first embodiment is used. At a predetermined position of the inner side-wall surface of the supporting portion 19a in the button receiving member 9B, a click projected portion 19ac, serving as an engaged portion, circumferentially-formed throughout the entire periphery is formed.

The operating button 3B is a substantially cylindrical member having the opening at one end. At the one end having the opening, a flange unit 3Bb is formed. At the bottom portion of the flange unit 3Bb, a click spring foot 3Bc, constituting with six engaging portions with the elasticity, is formed. Incidentally, the click spring foot 3Bc contains, e.g., thermoplastic resin (polypropylene), and is integrally formed to the operating button 3B.

The click spring foot 3Bc has a distal end which is wide to the outside so that it is wider than an outer-diameter dimension D6 of the operating button 3. An outer-diameter dimension D5 at the distal end is set to be slightly larger than an outer-diameter dimension D4 (refer to Fig. 6) at the top portion of the click projected portion 19ac of the button receiving member 9B. Therefore, upon moving the operating button 3B in the direction shown by an arrow X in Fig. 6, the click spring foot 3Bc comes into contact with the click projected portion 19ac of the button receiving member 9B and goes over the click projected portion 19ac, thereby forming the click mechanism for generating the sense of click operation.

The micro switch 20 is arranged at a predetermined position with which a predetermined portion of the distal end of the click spring foot 3Bc of the operating button 3B comes into contact. Thus, upon pressing the operating button 3B in the direction shown by the arrow X1 at the highest level, a switch portion of the micro switch 20 is pressed and the resultant generated signal is transmitted to the fluid control apparatus 11 (refer to Fig. 1).

Incidentally, the magnetic member 21 is integrally arranged to the operating button 3B, the distance between the magnetic member 21 and the magnetic sensor 22 changes in accordance with the movement of the operating button 3B in the direction shown by the arrow X. Therefore, the magnetic sensor 22 detects the level of the magnetic field of the magnetic member 21, thereby detecting the distance between the magnetic sensor 22 and the magnetic member 21, that is, positional information of the operating button 3B. The detecting result obtained by the magnetic sensor 22 is transmitted to the fluid control apparatus 11.

The operation of the fluid control input member 1B with the above-mentioned structure is as follows.

That is, the operator presses the operating button 3B, thereby moving the operating button 3B in the direction shown by an arrow X1 shown in Fig. 6. In accordance with the pressing operation of the operating button 3B, the bending of a rubber cover (not shown) starts. Then, the click spring foot 3Bc comes into contact with the click projected portion 19ac of the button receiving member 9B. Thereafter, the click spring foot 3Bc is modified toward the inside and goes over the click projected portions 19ac (in the state shown by a dotted line in Fig. 6).

Just before the timing at which the click spring foot 3Bc goes over the click projected portion 19ac, a rubber cover (not shown) is bent and is folded similarly to the case according to the first embodiment. Thus, the sense of click operation is generated.

Before the click spring foot 3Bc comes into contact with the click projected portions 19ac, the amount of pressing force of the operating button 3B is gradually increased by the operation when the rubber cover is bent. At the moment at which the rubber cover is folded, the amount of pressing force of the operating button 3B sharply reduces.

Thereafter, while the click spring foot 3Bc comes into contact with the click projected portion 19ac and goes over them, the elastic force of the click spring foot 3Bc gradually increases the amount of pressing force of the operating button 3B. When the click spring foot 3Bc goes over the click projected portion 19ac, the amount of pressing force of the operating button 3B sharply reduces at the timing. Thus, the sense of click operation is generated.

At the just-after timing, a predetermined portion of the operating button 3B presses the switch portion of the micro switch 20, thereby generating a predetermined signal from the micro switch 20. The generated signal is transmitted to the fluid control apparatus 11. In response to the transmitted signal, the fluid control apparatus 11 executes the switching control processing from the air supply operation to the water supply operation.

That is, according to the second embodiment, the pressing operation of the operating button 3B starts the air supply operation. In accordance with the pressing operation of the operating button 3B, the amount of air supplied increases. The amount of air supplied is increased at the timing before/after generating the sense of click operation when the rubber cover is folded at the timing before the click spring foot 3Bc comes into contact with the click projected portion 19ac.

Just after generating the sense of click operation when the click spring foot 3Bc goes over the click projected portion 19ac, the micro switch 20 operates, thereby switching the operation from the air supply operation to the water supply operation.

As mentioned above, according to the second embodiment, the same advantages as those according to the first embodiment are obtained.

Since a switching signal from the air supply operation to the water supply operation is generated by using the micro switch 20, the control for switching the operation is more certainly executed and this contributes to the reduction in parts.

According to the second embodiment, when the operating button 3B is moved in the direction shown by the arrow X1, the amount of air supplied is set to be maximum at the timing for generating the sense of click operation (first time) by using the rubber cover. The air supply operation is switched to the water supply operation at the timing for generating the sense of click operation (second time) by using the click mechanism (having the click spring foot 3Bc and the click projected portion 19ac). The operation starting at the timing for generating the sense of click operation is not limited to this and may be executed by combining other operations.

According to the second embodiment, the click projected portion 19ac is formed to the button receiving member 9B at only one position. In addition, for example, the similar-shaped projected portion is arranged so that the click spring foot 3Bc goes over the two click projected portions (19ac) two times upon moving the operating button 3B. Then, the sense of click operation is generated three times, including the sense of click operation when the rubber cover is folded upon moving the operating button 3B in the direction shown by the arrow X1 or in the direction shown by the arrow X2.

In this case, at the first timing for generating the sense of click operation (using the rubber cover), the amount of air supplied is set to be maximum. At the second timing of generating the sense of click operation (at the first time with the click mechanism), the spray operation starts. At the third timing for generating the sense of click operation (at the second time with the click mechanism), the operation is switched to the water supply operation.

In the case of starting the operation or switching the operation at the timing for generating the sense of click operation, the setting of the so-called dead zone is not necessary. Therefore, the entire pressing stoke of the operating button 3 is reduced. The dimension of the operating member in the height direction is suppressed and this contributes to the reduction in size in device.

In the case of arranging the two click projected portions (19ac) as mentioned above, the dimensions of the click projected portions 19ac in the height direction are different. Then, it is possible to increase/reduce the sense of click operation which is generated by the corresponding click projected portions 19ac. Therefore, the type of the previous operation is always recognized by the sense of click operation, and the next operation is easily grasped only by obtaining the operating sense.

Next, a description is given of a fluid control input device according to a third embodiment of the present invention

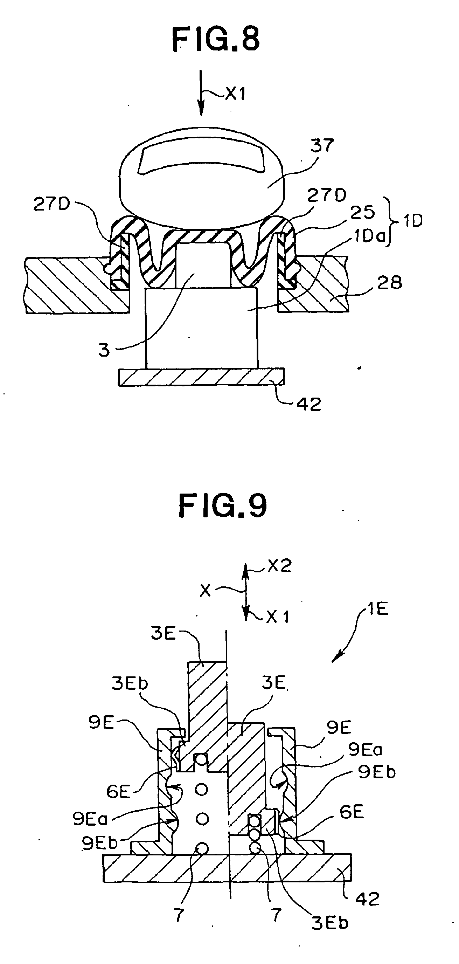

Fig. 9 is a sectional view showing the structure of a fluid control input device (fluid control input member 1E), showing the normal state before operating the operating button at the left half portion and the pressing state of the operating button at the right half portion, according to the fourth embodiment of the present invention.

The structure according to the third embodiment basically uses the structure according to the first embodiment. However, the operating button and the button receiving member for receiving the operating button and the click mechanism thereof are different from the structure according to the first embodiment. Therefore, the same components as those according to the first embodiment are designated by the same reference numerals and a detailed description thereof is omitted. Further, according to the third embodiment, a rubber cover for entirely covering the fluid control input device is arranged. The rubber cover according to the third embodiment is arranged similarly to that according to the first embodiment and is not shown for the purpose of a brief description of the drawing. An endoscope using the fluid control input device according to the third embodiment and a fluid control apparatus connected to the endoscope are the same as those according to the first embodiment, therefore, are referred to Fig. 1, and a drawing and a description thereof are omitted. Only the different portions are described hereinbelow.

Similarly to the first embodiment, the fluid control input member 1E according to the third embodiment comprises: a rubber cover (not shown); an operating button 3E; and a button receiving member 9E. The button receiving member 9E is fixed and supported by predetermined fixing means (not shown) at a predetermined position of the top surface of the fixing substrate 42.

A flange unit 3Eb is formed on the side of the bottom end of the operating button 3. Click springs 6E, serving as a plurality of (e.g., three) engaging portions, are integrally fixed and supported to be projected toward the outside at predetermined positions on the outer-circumference of the flange unit 3Eb.

The button receiving member 9E comprises, on the inner wall surface thereof, a click caved portion 9Ea (caved portion) and a click projected portion 9Eb (projected portion), serving as engaged portions, corresponding to the click springs 6E. The click caved portion 9Ea and the click projected portion 9Eb, serving as the engaged portions may be caved with the groove along the inner wall surface and be projected continuously arranged. Or, the click caved portion 9Ea and the click projected portion 9Eb may be formed only at predetermined portions corresponding to the moving range of the click springs 6E.

With the above-mentioned structure, in the movement of the operating button 3E in the direction shown by an arrow X in Fig. 9, the click springs 6E pass through the click caved portion 9Ea and then are fit into the click caved portion 9Ea, thereby instantaneously reducing the amount of pressing force of the operating button 3. Thereafter, the amount of pressing force of the operating button 3 increases, thereby pulling-out the click springs 6E from the click caved portion 9Ea. Thus, the sense of click operation is obtained.

When the click springs 6E pass through the click projected portion 9Eb, the click springs 6E are pressed to the click projected portion 9Eb, thereby gradually increasing the amount of pressing force of the operating button 3. Then, the click springs 6E go over the click projected portion 9Eb, thereby instantaneously reducing the amount of pressing force of the operating button 3. Thus, the sense of click operation is obtained.

Incidentally, referring to Fig. 9, the position detecting means for detecting the position or the amount of movement of the operating button 3E is the same as that according to the first embodiment and a drawing thereof is omitted. Other structures are the same as those according to the first embodiment.

The operation of the fluid control input member 1E according to the third embodiment with the above structure is as follows. That is, in the state at the left half portion in Fig. 9, the operating button 3E is pressed in the direction shown by an arrow X1 in Fig. 9. Then, the operating button 3E is moved in the same direction. Simultaneously, predetermined operation of the air supply operation, or the like, starts.

Then, the click springs 6E integrally-fixed at predetermined positions of the operating button 3E are moved along the inner wall surface of the button receiving member 9E and are then fit into the click caved portion 9Ea. In accordance with the movement of the operating button 3E, the operation is controlled to gradually increase the mount of fluid due to the air supply operation, or the like.

When the click springs 6E are fit into the click caved portion 9Ea, the amount of pressing force of the operating button 3E is instantaneously reduced. Further, the operating button 3E is pressed and the click springs 6E are then pulled-out from the click caved portion 9Ea. The amount of pressing force in this case slightly increases.

When the click springs 6E are fit into the click caved portion 9Ea, the operating button 3E is temporarily positioned. Incidentally, the elasticity of the repulsive spring 7 in the direction shown by the arrow X2 (extending direction) in this case operates to the operating button 3E. The elasticity is set to the amount of force for preventing the pull-out of the click springs 6 from the click caved portion 9Ea. Therefore, if the pressing force is released from the operating button 3E in this state, the state is kept.

Subsequently, the operating button 3E is further pressed in the direction shown by the arrow X1. Then, the click springs 6E are detached from the click caved portion 9Ea and are moved in the same direction. Here, the switching control operation may be executed from the air supply operation to the water supply operation. Subsequently, the operating button 3 is pressed in the same direction, the water supply operation then continues, and the amount of fluid gradually increases.

Then, the click springs 6E come into contact with the click projected portion 9Eb. From this state, the operating button 3E is further pressed and the amount of pressing force gradually increases. When the click springs 6E are arranged to the top of the click projected portion 9Eb, the necessary amount of pressing force is maximum. Further, the operating button 3 is pressed and the click springs 6E go then over the top of the operating button 3. Instantaneously, the amount of pressing force of the operating button 3 reduces. The state is shown at the right half portion in Fig. 9. In this state, similarly to the above case (just after the sense of click operation at the first time), in the click springs 6E, the click projected portion 9Eb regulates the elasticity due to the repulsive spring 7 in the direction shown by the arrow X2, thereby stopping the operating button 3E at the position.

As mentioned above, in the fluid control input member 1E, the sense of click operation two times is obtained within the moving range of the operating button 3E in the direction shown by the arrow X. At a predetermined timing before/after generating the sense of click operation two times, the control operation is performed to adjust the amount of fluid by the air supply operation or end the air supply operation and to start or end the water supply operation and adjust the amount of fluid by the water supply operation. In addition, when the rubber cover 25 is bent and is folded, the sense of click operation can be generated. Thus, the operability is ensured with more certainty and the erroneous operation is easily suppressed.

Incidentally, the positional relationship between the click caved portion 9Ea and the click projected portion 9Eb is not limited to this. For example, referring to Fig. 9, the click projected portion 9Eb may be formed on the top side and the click caved portion 9Ea may be formed on the bottom. Further, in place of the click projected portion 9Eb shown in Fig. 9, a concaved portion similar to the click caved portion 9Ea may be formed, thereby combining the concaved portions. On the other hand, in place of the click caved portion 9Ea shown in Fig. 9, a projected portion similar to the click projected portion 9Eb may be formed, thereby combining the projected portions.

Incidentally, the present invention is not limited to the above embodiments and can be modified without departing from the essentials of the present invention.

### Industrial Applicability

According to the present invention, as mentioned above, the fluid control input device for endoscope according to the present invention is preferably applied to a fluid control apparatus for an endoscope, which supplies the fluid and absorbs the intracavital tissue via a channel arranged in the endoscope.

## Claims

1. A fluid control input device (1) for endoscope comprising:
a moving member (3) that is moved in accordance with the operation for instructing the switching of a channel arranged in the endoscope by an operator;
a restoring member (7) that restores the moving member (3) to a predetermined restoring position;
an operated amount detecting member (2, 4) that detects at least one of the amount of movement of the moving member and the amount of operated force of the moving member by the operation of the operator and outputs information corresponding to at least one of the detected amount of movement and the detected amount of operated force;
a covering member (25) that is watertightly connected to an exterior member of the endoscope so as to watertightly cover the moving member (3) while permitting the movement of the moving member; and
an amount-of-force changing mechanism (6) that changes the amount of operated force of the moving member after/before a predetermined position at which the moving member is moved by the operation of the operator upon moving the moving member to the predetermined position, **characterized in that** the amount-of-force changing mechanism (6) comprises:
an engaging portion (3a) arranged to the moving member (3); and
a click mechanism (6a) that is arranged on a moving path of the engaging portion in accordance with the movement of the moving member, and changes the amount of operated force of the moving member by the engagement with the engaging portion.

2. A fluid control input device for endoscope according to Claim 1, wherein the covering member contains an elastically modifying member, and
the amount-of-force changing mechanism enables the covering member to be bent and generate the change in amount of force after/before the predetermined position.

3. A fluid control input device for endoscope according to Claim 1, wherein the click mechanism comprises a click spring.

4. A fluid control input device for endoscope according to Claim 1, wherein the click mechanism comprises a ball and a spring that presses the ball onto the moving path of the engaging portion.

5. A fluid control input device for endoscope according to Claim 2, wherein the amount-of-force changing mechanism comprises:
an engaging portion (3a) that is integrally arranged to the moving member (3) and contains an elastically modifying member; and
an engaged portion that is arranged onto the moving path of the engaging portion in accordance with the movement of the moving member, wherein
the engaging portion is engaged with the engaged portion, thereby changing the amount of operated force of the moving member.

6. A fluid control input device for endoscope according to Claim 5, wherein the engaged portion comprises a projected portion that is projected onto the moving path of the engaging portion.

7. A fluid control input device for endoscope according to Claim 6, wherein the engaged portion further comprises a caved portion that is evacuated from the moving path of the engaging portion.

8. A fluid control input device for endoscope according to Claim 1, wherein a plurality of the moving members are arranged, and
the covering member integrally covers the plurality of moving members.

9. A fluid control input device for endoscope according to Claim 1, wherein the covering member comprises a first shape bendable portion that is bent by a first amount of force of the elastically modifying member and a second shape bendable portion that is bent by a second amount of force larger than the first amount of force, and
the amount-of-force changing mechanism enables the covering member to generate a first change in amount of force by the operation that the first shape bendable portion is bent before the predetermined position, and to further generate a second change in amount of force by the operation that the second shape bendable portion is bent after the predetermined position of the covering member.

## Patentansprüche

1. Fluidsteuerungseingabeeinrichtung (1) für ein Endoskop, folgendes aufweisend:
ein bewegliches Bauteil (3), welches entsprechend der Betätigung durch eine Bedienungsperson zur Anweisung des Wechsels eines im Endoskop angeordneten Kanals bewegt wird;
ein Rücksetzteil (7), welches das bewegliche Bauteil (3) in eine vorgegebene Rücksetzposition rückführt;
einen Detektor (2, 4) für den Betätigungsbetrag, der zumindest den Betrag der Bewegung des beweglichen Teils oder den Betrag der Betätigungskraft der Bedienungsperson bezüglich des beweglichen Teils detektiert und Information ausgibt entsprechend zumindest des ermittelten Betrags der Bewegung oder des ermittelten Betrags der Betätigungskraft;
ein Abdeckteil (25), welches wasserdicht mit einem äußeren Bauteil des Endoskops verbunden ist, sodass das bewegliche Teil (3) wasserdicht abgedeckt ist und die Bewegung des beweglichen Teils ermöglicht ist; und
eine Wechseleinrichtung (6) bezüglich des Betrages der Kraft, welche den Betrag der Betätigungskraft des beweglichen Teils nach/vor einer vorgegebenen Position ändert, bei der das bewegliche Teil durch die Betätigung seitens der Bedienungsperson zum Bewegen des beweglichen Teils bewegt wird, **dadurch gekennzeichnet, dass** die Wechseleinrichtung (6) zum Ändern des Betrages der Kraft folgendes aufweist:
einen Eingriffsabschnitt (3a), der in Bezug auf das bewegliche Teil (3) angeordnet ist; und
einen Rastmechanismus (6a), der auf einer Wegstrecke des Eingriffsabschnitts entsprechend der Bewegung des beweglichen Teils angeordnet ist und den Betrag der Betätigungskraft des beweglichen Teils durch Eingriff mit dem Eingriffsabschnitt ändert.

2. Fluidsteuerungseingabeeinrichtung gemäß Anspruch 1, wobei das Abdeckteil ein elastisch modifizierendes Teil enthält und der Wechselmechanismus für den Betrag der Kraft ermöglicht, dass das Abdeckteil gebogen werden kann und den Wechsel in dem Betrag der Kraft nach/vor der vorgegebenen Position erzeugt.

3. Fluidsteuerungseingabeeinrichtung gemäß Anspruch 1, wobei der Rastmechanismus eine Rastfeder aufweist.

4. Fluidsteuerungseingabeeinrichtung gemäß Anspruch 1, wobei der Rastmechanismus eine Kugel und eine Feder aufweist, welche die Kugel in den Bewegungsweg des Eingriffsabschnitts drängt.

5. Fluidsteuerungseingabeeinrichtung gemäß Anspruch 2, wobei der Wechselmechamismus für den Betrag der Kraft folgendes aufweist:
einen Eingriffsabschnitt (3a), der integral mit dem beweglichen Teil (3) ausgebildet ist und ein elastisches Modifizierungsteil aufweist; und
einen in Eingriff stehenden Abschnitt, der auf dem Wegstück des Eingriffsabschnitts entsprechend der Bewegung des beweglichen Teils angeordnet ist, wobei
der Eingriffsabschnitt mit dem in Eingriff stehenden Abschnitt wechselwirkt, wodurch der Betrag der Betätigungskraft des beweglichen Teils geändert wird.

6. Fluidsteuerungseingabeeinrichtung gemäß Anspruch 5, wobei der in Eingriff stehende Abschnitt einen Vorsprung aufweist, der in die Bewegungsstrecke des Eingriffsabschnittes ragt.

7. Fluidsteuerungseingabeeinrichtung für ein Endoskop gemäß Anspruch 6, wobei der in Eingriff stehende Abschnitt weiterhin einen ausgehöhlten Abschnitt aufweist, der vom Bewegungsweg des Eingriffsabschnittes evakuiert ist.

8. Fluidsteuerungseingabeeinrichtung für ein Endoskop gemäß Anspruch 1, wobei eine Mehrzahl von beweglichen Teilen vorgesehen ist und das Abdeckteil die Mehrzahl beweglicher Teile integral abdeckt.

9. Fluidsteuerungseingabeeinrichtung für ein Endoskop gemäß Anspruch 1, wobei das Abdeckteil einen biegbaren Abschnitt erster Form aufweist, der durch einen ersten Betrag an Kraft des elastisch modifizierenden Teils biegbar ist, und einen biegbaren Abschnitt zweiter Form, der durch einen zweiten Betrag an Kraft biegbar ist, die größer ist als der erste Betrag an Kraft; und
der Wechselmechanismus bezüglich des Betrags der Kraft das Abdeckteil instand versetzt, einen ersten Wechsel bezüglich des Betrags der Kraft zu erzeugen **dadurch**, dass der biegbare Abschnitt erster Form vor der vorgegebenen Stellung gebogen ist, und dass weiterhin ein zweiter Wechsel im Betrag der Kraft **dadurch** erzeugt wird, dass der biegbare Abschnitt zweiter Form nach der vorgegebenen Stellung des Abdeckteils gebogen ist.

## Revendications

1. Dispositif d'entrée de commande de fluide (1) pour endoscope comprenant :
un élément mobile (3) qui est déplacé en fonction de l'opération pour ordonner la commutation d'un canal agencé dans l'endoscope par un opérateur ;
un élément de restauration (7) qui restaure l'élément mobile (3) à une position de restauration prédéterminée ;
un élément de détection de quantité opérée (2, 4) qui détecte au moins l'une de la quantité de mouvement de l'élément mobile et de la quantité de force opérée de l'élément mobile par l'opération de l'opérateur et délivre en sortie des informations correspondant à au moins l'une de la quantité détectée de mouvement et de la quantité détectée de force opérée ;
un élément de recouvrement (25) qui est connecté de façon étanche à l'eau à un élément extérieur de l'endoscope de manière à recouvrir de façon étanche à l'eau l'élément mobile (3) tout en permettant le mouvement de l'élément mobile ; et
un mécanisme de changement de quantité de force (6) qui change la quantité de force opérée de l'élément mobile après/avant une position prédéterminée à laquelle l'élément mobile est déplacé par l'opération de l'opérateur au déplacement de l'élément mobile jusqu'à la position prédéterminée, **caractérisé en ce que** le mécanisme de changement de quantité de force (6) comprend :
une partie d'engagement (3a) agencée sur l'élément mobile (3) ; et
un mécanisme de cliquet (6a) qui est agencé sur un chemin de déplacement de la partie d'engagement en fonction du mouvement de l'élément mobile, et change la quantité de force opérée de l'élément mobile par l'engagement avec la partie d'engagement.

2. Dispositif d'entrée de commande de fluide pour endoscope selon la revendication 1, dans lequel l'élément de recouvrement contient un élément de modification élastique, et
le mécanisme de changement de quantité de force permet que l'élément de recouvrement soit plié et génère le changement de la quantité de force après/avant la position prédéterminée.

3. Dispositif d'entrée de commande de fluide pour endoscope selon la revendication 1, dans lequel le mécanisme de cliquet comprend un ressort de cliquet.

4. Dispositif d'entrée de commande de fluide pour endoscope selon la revendication 1, dans lequel le mécanisme de cliquet comprend une bille et un ressort qui presse la bille sur le chemin de déplacement de la partie d'engagement.

5. Dispositif d'entrée de commande de fluide pour endoscope selon la revendication 2, dans lequel le mécanisme de changement de quantité de force comprend :
une partie d'engagement (3a) qui est agencée de façon intégrale sur l'élément mobile (3) et contient un élément de modification élastique ; et
une partie engagée qui est agencée sur le chemin de déplacement de la partie d'engagement en fonction du mouvement de l'élément mobile, dans lequel
la partie d'engagement est engagée avec la partie engagée, changeant ainsi la quantité de force opérée de l'élément mobile.

6. Dispositif d'entrée de commande de fluide pour endoscope selon la revendication 5, dans lequel la partie engagée comprend une partie projetée qui est projetée sur le chemin de déplacement de la partie d'engagement.

7. Dispositif d'entrée de commande de fluide pour endoscope selon la revendication 6, dans lequel la partie engagée comprend en outre une partie affaissée qui est évacuée du chemin de déplacement de la partie d'engagement.

8. Dispositif d'entrée de commande de fluide pour endoscope selon la revendication 1, dans lequel une pluralité d'éléments mobiles sont agencés, et
l'élément de recouvrement recouvre de façon intégrale la pluralité d'éléments mobiles.

9. Dispositif d'entrée de commande de fluide pour endoscope selon la revendication 1, dans lequel l'élément de recouvrement comprend une partie pliable d'une première forme qui est pliée par une première quantité de force de l'élément de modification élastique et une partie pliable d'une deuxième forme qui est pliée par une deuxième quantité de force plus grande que la première quantité de force, et
le mécanisme de changement de quantité de force permet que l'élément de recouvrement génère un premier changement de la quantité de force par l'opération que la partie pliable d'une première forme est pliée avant la position prédéterminée, et génère en outre un deuxième changement de la quantité de force par l'opération que la partie pliable d'une deuxième forme est pliée après la position prédéterminée de l'élément de recouvrement.
